# EUROPEAN PATENT SPECIFICATION

(11) **EP 2 246 000 B1**
(45) Date of publication and mention of the grant of the patent: **06.06.2012**
(21) Application number: 09010620.4
(22) Date of filing: 18.08.2009
(51) Int. Cl.: A61B 17/28, A61B 19/00, A61M 1/00

(54) **Surgical instrument**
Chirurgisches Instrument
Instrument chirurgical

(30) Priority: 27.04.2009 KR 20090036479
(43) Date of publication of application: 03.11.2010
(73) Proprietor: National Cancer Center, Ilsandong-gu Goyang-si, Gyeonggi-do (KR)
(72) Inventor: Cho, Seong-Yeon, Goyang-si Gyeonggi-do (KR); Park, Sang-Jae, Goyang-si Gyeonggi-do (KR); Kim, Kwang-Gi, Goyang-si Gyeonggi-do (KR)
(74) Representative: Schoppe, Fritz

(56) References cited:
- US-A- 3 980 086
- US-A- 5 147 356
- US-A- 5 727 569
- US-B1- 6 185 356

## Description

### BACKGROUND

### 1. Technical Field

Example embodiments of the present invention relates to surgical instrument, and more particularly, to surgical instrument used in surgery for dissecting body tissue of a patient.

### 2. Discussion of Related Art

During surgery, an operator may dissect soft tissue of a patient to obtain a desired surgical field. However, since live body tissue receives blood supply, bleeding may occur in the dissected tissue. In order to stop the bleeding, an assistant may rapidly remove the already issued blood and ongoing bleeding with a suction device so that the operator can identify the bleeding point and control the bleeding. If the bleeding is controlled, the operator performs further dissection of deeper tissue to enter a desired surgical site. When the bleeding occurs again in deeper tissue, the operator and the assistant repeat the same procedure. As the depth of the dissection plane is increased, the operator and the assistant encounter poor illumination conditions as well as the repeated bleeding in deeper tissue. The reason for this is that when the surgical site is deepened, the light of an astral lamp hanging from the ceiling of an operating room does not reach the deep surgical site. As a result, when the depth of the dissection plane is increased, the operator may face difficulties in performing the surgery due to the bleeding from deeper tissue and poorer illumination conditition. As it is difficult to stop the bleeding rapidly due to such reasons, the amount of surgical bleeding and the operating time may increase. Furthermore, due to the poor field of view, other important internal organs, which are unrelated to the purpose of the surgery, may be damaged. Finally, due to these reasons, post-surgical morbidity or mortality rates could be increased.

US 3980086 A discloses a surgical instrument of scissors configuration. The instrument comprises a pair of elongated members which are pivotally connected. Functional ends of the members are provided with serrated inner faces and bores are opening into one of the inner faces. A fluid conduit is formed within the member to provide fluid communication with the bores. A projecting end portion of the conduit is adapted to permit connection with a vacuum source.

US 6185356 B1 discloses a protective cover for protecting a lighting device from direct contact with contaminants or components which may interfere with proper operation thereof. The contaminant (e.g., blood, body tissue, dirt, oil, grease, paint, etc.) or other component (e.g., adhesive pad) can interfere with the desired internal reflection of the light propogating through a light transmitting member, by changing the angle of reflection of the propagating light. Forceps having an integrated light delivery system are discloses.

### SUMMARY OF THE INVENTION

Example embodiments of the present invention are directed to surgical instruments which can provide a field of view over a surgical site by sucking blood from a bleeding site caused in a dark and deep surgical site and illuminating the surgical site during surgery on body tissue of a patient, thus allowing an operator to safely and rapidly perform the surgery.

Additional aspects of example embodiments of the invention will be set forth in the description which follows, and in part will be apparent from the description, or may be learned by practice of the invention.

In example embodiments, a surgical instrument includes: a first body including at least one suction port provided at a front end thereof; a second body configured to cross the first body and to be rotatably coupled to the first body; a suction unit provided in the first body to suck blood from a bleeding site of a patient through the suction port during surgery; and an illuminator provided in the second body to illuminate a space between front ends of the first and second bodies.

The suction unit may include a suction tube mounted in the first body to be coupled to the suction port.

A suction tube insertion hole may be formed in the first body in the longitudinal direction such that the suction tube can be inserted and mounted in the suction tube insertion hole.

The suction port may have a diameter smaller than the suction tube insertion hole.

The suction port may be formed on a front surface of a front end or on an inner surface of the first body.

The illuminator may include an optical fiber mounted in the second body and configured to guide light generated from a light source to be irradiated on a space between the front ends of the first and second bodies. Here, the light source may be a light emitting diode (LED).

An optical fiber insertion hole may be formed in the second body in the longitudinal direction such that the optical fiber can be inserted and mounted in the optical fiber insertion hole.

At least one illumination hole, through which the front end of the optical fiber is exposed, may be formed in the second body, and the illumination hole may be formed on an inner surface spaced a predetermined distance from the front end of the second body to the rear.

The optical fiber exposed through the illumination hole may be arranged to illuminate the central area between the opened front ends of the first and second bodies.

The illuminator may be a light bulb or an LED lamp provided on an inner surface spaced a predetermined distance from the front end of the second body to the rear so as to illuminate the central area between the opened front ends of the first and second bodies.

The front ends of the first and second bodies may be bent upward or downward.

Handles may be formed on the rear ends of the first and second bodies such that a user's fingers are inserted into the handles to hold the surgical instrument.

The first and second bodies may be rotatably coupled to each other by a rotational connector. Here, the rotational connector may include: a first rotational connector integrally formed in the center of the first body and including a pair of coupling protrusions formed at the edge of one side of the first body; and a second rotational connector integrally formed in the center of the second body and including a pair of connecting grooves formed at the edge of one side opposite to the first rotational connector such that the pair of coupling protrusions are inserted into the pair of connecting grooves and the first rotational connector and the second rotational connector rotate with respect to each other at a predetermined rotational angle.

In other example embodiments, a surgical instrument may include: a first body including at least one suction port provided at a front end thereof; a second body configured to cross the first body and to be rotatably coupled to the first body and including at least one illumination hole formed at a front end thereof; a suction tube mounted in the first body to suck blood from a bleeding site of a patient through the suction port during surgery; an optical fiber mounted in the second body and configured to illuminate a space between the front ends of the first and second bodies; a suction device configured to provide a suction force to the suction tube; and a light source configured to provide light to the optical fiber.

### BRIEF DESCRIPTION OF THE DRAWINGS

The above and other objects, features and advantages of example embodiments of the present invention will become more apparent to those of ordinary skill in the art by describing in detail example embodiments thereof with reference to the attached drawings, in which:
FIG. 1 is a perspective view schematically showing a surgical instrument in accordance with an example embodiment of the present invention;
FIG. 2 is an exploded perspective view of the surgical instrument of FIG. 1;
FIG. 3 is a side view of a first body of FIG. 1;
FIG. 4 is a side view of a second body of FIG. 1;
FIG. 5 is an enlarged perspective view as viewed from the rear side of portion A of FIG. 1;
FIG. 6 is an enlarged perspective view of portion B of FIG. 1;
FIG. 7 is a perspective view showing another example of portion B of FIG. I
FIG. 8 is an enlarged perspective view as viewed from the rear side of portion C of FIG. 1; and
FIG. 9 is an exemplary diagram illustrating use of the surgical instrument in accordance with an example embodiment of the present invention.

### DETAILED DESCRIPTION OF EXAMPLE EMBODIMENTS

Hereinafter, example embodiments of the present invention will be described in detail. However, the present invention is not limited to the embodiments disclosed below, but can be implemented in various forms. The following embodiments are described in order to enable those of ordinary skill in the art to embody and practice the present invention.

Advantages and features of the present invention and methods of accomplishing the same may be understood more readily by reference to the following detailed description of example embodiments and the accompanying drawings. The present invention may, however, be embodied in many different forms and should not be construed as being limited to the example embodiments set forth herein. Rather, these example embodiments are provided so that this disclosure will be thorough and complete and will fully convey the concept of the present invention to those skilled in the art, and the present invention will only be defined by the appended claims. Like reference numerals refer to like elements throughout the specification.

Hereinafter, a surgical instrument in accordance with an example embodiment of the present invention will be described in detail with reference to the accompanying drawings. In the following description, detailed description of well-known functions or constructions that would obscure the invention in unnecessary detail will be omitted.

The term "dissection" used throughout the specification means an action of opening the space between laminated tissues or the space between heterogeneous tissues. Although the example embodiment illustrates a surgical instrument in the form of a body tissue dissector that enters the space between the laminated tissues or the space between heterogeneous tissues and opens the space, the present invention is not limited thereto and may be applied to various types of surgical instruments having shapes other than the dissector.

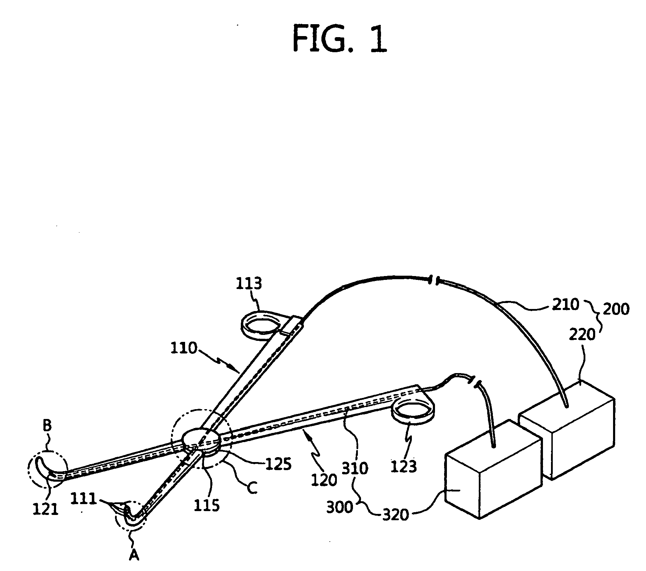

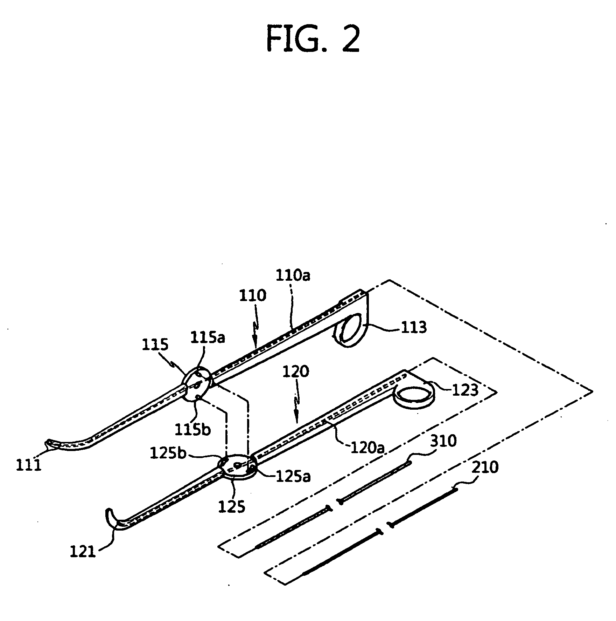

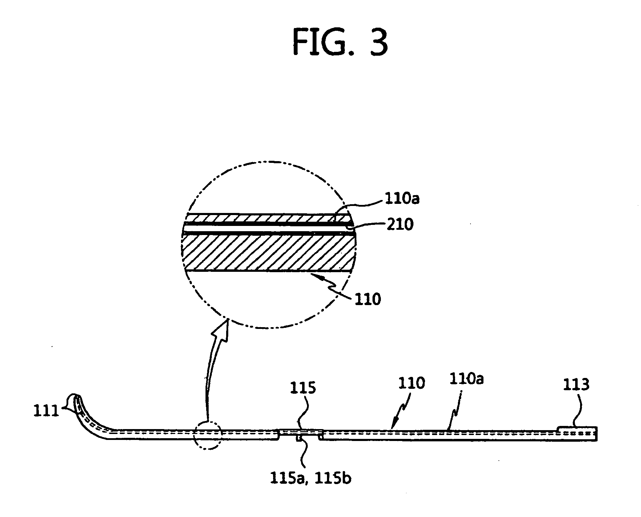

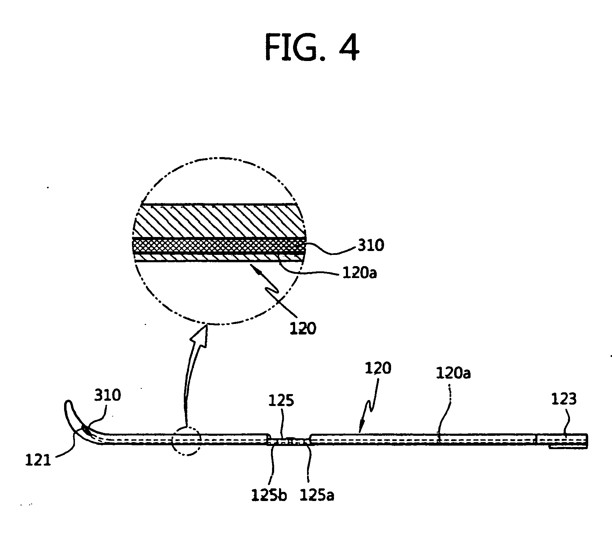

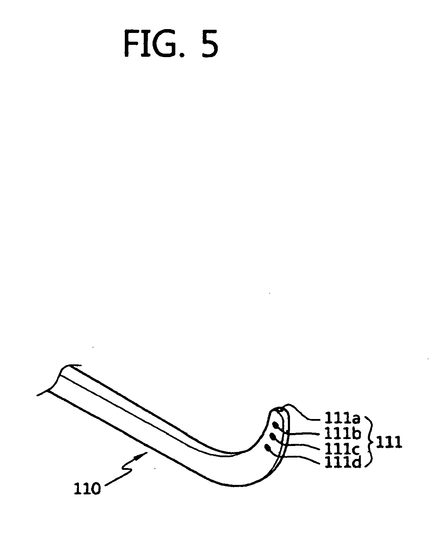

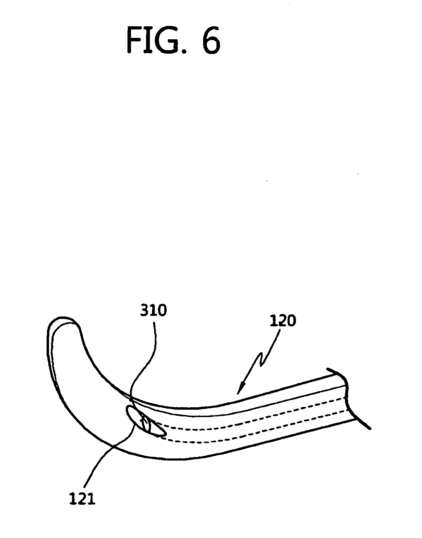

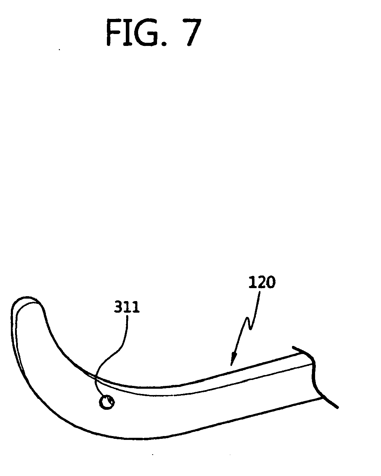

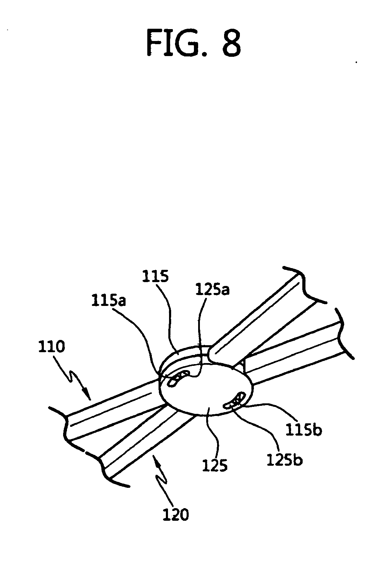

FIG. 1 is a perspective view schematically showing a surgical instrument in accordance with an example embodiment of the present invention, FIG. 2 is an exploded perspective view of the surgical instrument of FIG. 1, FIGS. 3 and 4 are side views of a first body and a second body of FIG. 1, FIG. 5 is an enlarged perspective view as viewed from the rear side of portion A of FIG. 1, FIG. 6 is an enlarged perspective view of portion B of FIG. 1, FIG. 7 is a perspective view showing another example of portion B of FIG. 1, and FIG. 8 is an enlarged perspective view as viewed from the rear side of portion C of FIG. 1.

As shown in FIGS. 1 to 8, the surgical instrument in accordance with an example embodiment of the present invention may include a first body 110, a second body 120, a suction unit 200, and an illuminator 300.

The surgical instrument of an example embodiment of the present invention may be configured as a tissue dissector having substantially the same shape as a pair of scissors.

The first body 110 includes at least one suction port 111 provided at a front end thereof to suck blood or foreign materials. For example, although the example embodiment illustrates a configuration in which one suction port 111a is formed on a front surface of the front end of the first body 110 and two or three suction ports 111b, 111c, and 111d are formed on an inner surface thereof, the present invention is not limited thereto, and the suction port 111 may have various numbers and shapes. The suction port 111 may be formed to communicate with a suction tube insertion hole 110a, which will be described later, and also connected to a front end of a suction tube 210, which will also be described later.

The suction tube insertion hole 110a may be formed in the first body 110 in the longitudinal direction such that the suction tube 210 can be inserted and mounted in the suction tube insertion hole 110a. The example embodiment illustrates a configuration in which the suction tube insertion hole 110a is formed in the entire first body 110 in the longitudinal direction. However, the suction tube insertion hole 110a may be configured to penetrate the center of the first body 110 from the front end of the first body 110 to a first rotational connector 115, which will be described later, such that the suction tube 210 can be inserted and completely mounted in the suction tube insertion hole 110a. Further, a suction tube fixing groove (not shown) in the form of a semispherical groove may be formed on a side of the first body 110 from the first rotational connector 115 to a rear end of the first body 110 such that the radial portion of the suction tube 210 can be inserted and fixed to the suction tube fixing groove. Moreover, in order to prevent the blood or foreign materials sucked into the suction tube insertion hole 110a through the suction port 111 from being caught, the suction tube insertion hole 110a may have a greater diameter than the suction port 111. Furthermore, an inner diameter of the suction tube insertion hole 110a may increase from the front end of the first body 110 toward the rear.

The second body 120 may be configured to cross the first body 110 and to be rotatably coupled thereto by rotational connectors 115 and 125, which will be described later.

An optical fiber insertion hole 120a may be formed in the second body 120 in the longitudinal direction such that an optical fiber 310, which will be described later, can be inserted and mounted in the optical fiber insertion hole 120a. The example embodiment illustrates a configuration in which the optical fiber insertion hole 120a is formed in the entire second body 120 in the longitudinal direction. However, the optical fiber insertion hole 120a may be configured to penetrate the center of the second body 120 from an illumination hole 121 of the second body 120, which will be described later, to the second rotational connector 125, which will also be described later, such that the optical fiber 310 can be inserted and completely mounted in the optical fiber insertion hole 120a. Further, an optical fiber fixing groove (not shown) in the form of a semispherical groove may be formed on a side of the second body 120 from the second rotational connector 125 to the rear end of the second body 120 such that the radial portion of the optical fiber 310 can be inserted and fixed to the optical fiber fixing groove.

At least one illumination hole 121 coupled to the optical fiber insertion hole 120a and exposing the front end of the optical fiber 310 may be formed on the second body 120. Here, the illumination hole 121 may be formed on an inner surface spaced a predetermined distance from the front end of the second body 120 to the rear such that the optical fiber 310 can illuminate the central area between the opened front ends of the first and second bodies 110 and 120.

The front ends of the first and second bodies 110 and 120 may be bent upward or downward so as to easily open the space between tissues. Moreover, handles 113 and 123 in the form of rings may be formed at the rear ends of the first and second bodies 110 and 120 such that an operator's fingers can be inserted therethrough to hold the surgical instrument.

The first and second bodies 110 and 120 may be rotatably coupled to each other by the rotational connectors 115 and 125. The rotational connectors 115 and 125 may be coupled in a mutually rotatable manner.

The first rotational connector 115 may be integrally formed in substantially the center of the first body 110 in the form of a circle and include a pair of coupling protrusions 115a and 115b formed at edges of one side thereof.

The second rotational connector 125 may be integrally formed in substantially the center of the second body 120 in the form of a circle and include a pair of connecting grooves 125a and 125b formed at edges of one side opposite to the first rotational connector 115 such that the pair of coupling protrusions 115a and 115b can be inserted into the pair of connecting grooves 125a and 125b. As a result, the first rotational connector 115 and the second rotational connector 125 rotate with respect to each other at a predetermined angle, e.g., within a range of 0 to 30 degrees.

The suction unit 200 may be provided in the first body 110 so as to suck blood from a bleeding site or foreign materials generated during surgery such as tissue dissection through the suction port 111.

The suction unit 200 may include the suction tube 210 in the form of a tube inserted and mounted in the first body 110 such that its front end can be coupled to the suction port 111. The suction tube 210 may be inserted into the suction tube insertion hole 110a formed in the first body 110 in the longitudinal direction, in which its front end is coupled to the suction port 111 and its rear end is coupled to a conventional suction device 220 configured to provide a suction force to the suction tube 210.

The illuminator 300 may be provided in the second body 120 so as to illuminate the space between the opened front ends of the first and second bodies 110 and 120.

The illuminator 300 may include the optical fiber 310 inserted and mounted in the second body 120 and configured to guide light generated from a light source to be irradiated on the space between the opened front ends of the first and second bodies 110 and 120. Here, the light source 320 may include various types of light generating devices capable of providing light to the optical fiber 310, and preferably may be a light emitting diode (LED). The optical fiber 310 may be inserted into the optical fiber insertion hole 120a formed in the second body 120, in which its front end is exposed through the illumination hole 121 of the second body 120 and its rear end is coupled to the light source 320. The optical fiber 310 exposed through the illumination hole 121 may be arranged to illuminate the central area between the opened front ends of the first and second bodies 110 and 120.

Although the example embodiment illustrates a configuration in which the illuminator 300 includes the optical fiber 310, the present invention is not limited thereto, and the illuminator 300 may include a light bulb or LED lamp 311 provided on the inner surface spaced a predetermined distance from the front end of the second body 120 to the rear so as to illuminate the central area between the opened front ends of the first and second bodies 110 and 120, as shown in FIG. 7.

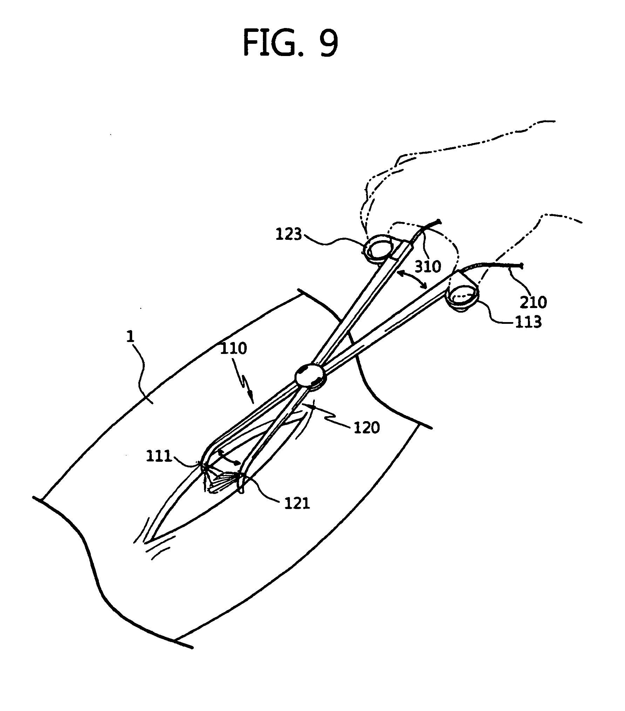

Next, the operation of the surgical instrument in accordance with an example embodiment of the present invention will be described in detail with reference to FIG. 9.

FIG. 9 is an exemplary diagram illustrating use of the surgical instrument in accordance with an example embodiment of the present invention.

As shown in FIG. 9, an operator inserts his or her fingers into the handles 113 and 123 of the surgical instrument to hold the surgical instrument and inserts the surgical instrument into a patient's body. At this time, the operator performs tissue dissection, in which the space between tissues is opened while both front ends of the surgical instrument are facing downward, and enters a desired surgical site. Here, bleeding occurs in the dissected tissue 1 during the tissue dissection. Therefore, the blood from the bleeding site and foreign materials generated during surgery are sucked and removed through the suction port 111 using the suction tube 210 mounted in the first body 110 of the surgical instrument in accordance with the present invention, thereby obtaining a surgical field of view.

When the operator inserts the surgical instrument deeper into the tissue to a desired surgical site, the depth of the dissected tissue is increased, and thus the surgical site becomes darker. As a result, it is difficult for the operator to obtain a better surgical field of view. At this time, light generated from the light source 320 is transmitted to the surgical area using the optical fiber 310 mounted in the second body 120 of the surgical instrument according to an example embodiment of the present invention so as to illuminate the central area between the opened front ends of the first and second bodies 110 and 120, thereby obtaining a surgical field of view.

As described above, according to a surgical instrument according to an example embodiment of the present invention, it is possible to provide a field of view over a surgical site by sucking blood from a bleeding site during surgery such as tissue dissection and illuminating a dark and deep surgical site, thus allowing the operator to safely and rapidly perform the surgery.

Moreover, example embodiments the present invention can provide convenience of use by configuring a small-sized surgical instrument to perform the functions of tissue dissection, blood suction, and illumination simultaneously.

While the example embodiments of the present invention have been described with reference to the accompanying drawings, it will be apparent to those skilled in the art that various modifications and variations can be made in the present invention without departing from the scope of the invention. Thus, it is intended that any future modifications of the embodiments of the present invention will come within the scope of the appended claims and their equivalents.

## Claims

1. A surgical instrument comprising:
a first body (110) including at least one suction port (111) provided at a front end thereof;
a second body (120) configured to cross the first body (110) and to be rotatably coupled to the first body (110);
a suction unit (200) provided in the first body (110) to suck blood from a bleeding site of a patient through the suction port (111) during surgery; and
an illuminator (300) provided in the second body (120) to illuminate a space between front ends of the first and second bodies (110, 120),
wherein the suction unit (200) comprises a suction tube (210) mounted in the first body (110) to be coupled to the suction port (111).

2. The surgical instrument of claim 1, wherein a suction tube insertion hole (110a) is formed in the first body (110) in the longitudinal direction such that the suction tube (210) is inserted and mounted in the suction tube insertion hole (110a).

3. The surgical instrument of claim 2, wherein the suction port (111) has a diameter smaller than the suction tube insertion hole (110a).

4. The surgical instrument of claim 1, wherein the suction port (111) is formed on a front surface of the front end or on an inner surface of the first body (110).

5. The surgical instrument of claim 1, wherein the illuminator (300) comprises an optical fiber (310) mounted in the second body (120) and configured to guide light generated from a light source to be irradiated on the space between the front ends of the first and second bodies (110, 120).

6. The surgical instrument of claim 5, wherein an optical fiber insertion hole (120a) is formed in the second body (120) in the longitudinal direction such that the optical fiber (310) is inserted and mounted in the optical fiber insertion hole (120a).

7. The surgical instrument of claim 5, wherein at least one illumination hole (121), through which a front end of the optical fiber (310) is exposed, is formed in the second body (120).

8. The surgical instrument of claim 7, wherein the illumination hole (121) is formed on an inner surface spaced a predetermined distance from the front end of the second body (120) to the rear.

9. The surgical instrument of claim 7, wherein the optical fiber (310) exposed through the illumination hole (121) is arranged to illuminate the central area between the opened front ends of the first and second bodies (110, 120).

10. The surgical instrument of claim 1, wherein the illuminator is a light bulb or an LED lamp provided on an inner surface spaced a predetermined distance from the front end of the second body (120) to the rear so as to illuminate the central area between the opened front ends of the first and second bodies (110, 120).

11. The surgical instrument of claim 1, wherein the front ends of the first and second bodies (110, 120) are bent upward or downward.

12. The surgical instrument of claim 1, wherein handles (113, 123) are formed on rear ends of the first and second bodies (110, 120) such that a user's fingers are inserted into the handles (113, 123) to hold the surgical instrument.

13. The surgical instrument of claim 1, wherein the first and second bodies (110, 120) are rotatably coupled to each other by a rotational connector (115, 125).

14. The surgical instrument of claim 13, wherein the rotational connector (115, 125) comprises:
a first rotational connector (115) integrally formed in the center of the first body (110) and including a pair of coupling protrusions (115a, 115b) formed at the edge of one side of the first body (110); and
a second rotational connector (125) integrally formed in the center of the second body (120) and including a pair of connecting grooves (125a, 125b) formed at the edge of one side opposite to the first rotational connector (115) such that the pair of coupling protrusions (115a, 115b) are inserted into the pair of connecting grooves (125a, 125b) and the first rotational connector (115) and the second rotational connector (125) rotate with respect to each other at a predetermined rotational angle.

## Patentansprüche

1. Ein chirurgisches Instrument, das folgende Merkmale aufweist:
einen ersten Körper (110), der zumindest einen ersten Saugmund (111), der an einem vorderen Ende desselben vorgesehen ist, umfasst;
einen zweiten Körper (120), der dazu konfiguriert ist, den ersten Körper (110) zu kreuzen und drehbar mit dem ersten Körper (110) gekoppelt zu sein;
eine Saugeinheit (200), die in dem ersten Körper (110) vorgesehen ist, um während einer Operation durch den Saugmund (111) Blut aus einer Blutungsstelle eines Patienten zu saugen; und
eine Beleuchtungsvorrichtung (300), die in dem zweiten Körper (120) vorgesehen ist, um einen Raum zwischen vorderen Enden des ersten und des zweiten Körpers (110, 120) zu beleuchten,
wobei die Saugeinheit (200) eine Saugkanüle (210) aufweist, die in dem ersten Körper (110) angebracht ist, um mit dem Saugmund (111) gekoppelt zu werden.

2. Das chirurgische Instrument gemäß Anspruch 1, bei dem ein Saugkanüleneinführungsloch (110a) in dem ersten Körper (110) in der Längsrichtung derart gebildet ist, dass die Saugkanüle (210) in das Saugkanüleneinführungsloch (110a) eingeführt und in demselben angebracht wird.

3. Das chirurgische Instrument gemäß Anspruch 2, bei dem der Saugmund (111) einen kleineren Durchmesser aufweist als das Saugkanüleneinführungsloch (110a).

4. Das chirurgische Instrument gemäß Anspruch 1, bei dem der Saugmund (111) auf einer vorderen Oberfläche des vorderen Endes oder auf einer Innenoberfläche des ersten Körpers (110) gebildet ist.

5. Das chirurgische Instrument gemäß Anspruch 1, bei dem die Beleuchtungsvorrichtung (300) eine optische Faser (310) aufweist, die in dem zweiten Körper (120) angebracht und dazu konfiguriert ist, aus einer Lichtquelle erzeugtes Licht dahin gehend zu lenken, dass es auf den Raum zwischen den vorderen Enden des ersten und des zweiten Körpers (110, 120) gestrahlt wird.

6. Das chirurgische Instrument gemäß Anspruch 5, bei dem ein Optische-Faser-Einführungsloch (120a) in dem zweiten Körper (120) in der Längsrichtung derart gebildet ist, dass die optische Faser (310) in das Optische-Faser-Einführungsloch (120a) eingeführt und in demselben angebracht wird.

7. Das chirurgische Instrument gemäß Anspruch 5, bei dem zumindest ein Beleuchtungsloch (121), durch das ein vorderes Ende der optischen Faser (310) belichtet wird, in dem zweiten Körper (120) gebildet ist.

8. Das chirurgische Instrument gemäß Anspruch 7, bei dem das Beleuchtungsloch (121) auf einer Innenoberfläche gebildet ist, die einen vorbestimmten Abstand von dem vorderen Ende des zweiten Körpers (120) nach hinten aufweist.

9. Das chirurgische Instrument gemäß Anspruch 7, bei dem die optische Faser (310), die durch das Beleuchtungsloch (121) belichtet wird, dahin gehend angeordnet ist, den mittleren Bereich zwischen den geöffneten vorderen Enden des ersten und des zweiten Körpers (110, 120) zu beleuchten.

10. Das chirurgische Instrument gemäß Anspruch 1, bei dem die Beleuchtungsvorrichtung eine Glühbirne oder eine LED-Lampe ist, die auf einer Innenoberfläche vorgesehen ist, die einen vorbestimmten Abstand von dem vorderen Ende des zweiten Körpers (120) nach hinten aufweist, um den mittleren Bereich zwischen den geöffneten vorderen Enden des ersten und des zweiten Körpers (110, 120) zu beleuchten.

11. Das chirurgische Instrument gemäß Anspruch 1, bei dem die vorderen Enden des ersten und des zweiten Körpers (110, 120) nach oben oder nach unten gebogen sind.

12. Das chirurgische Instrument gemäß Anspruch 1, bei dem Griffe (113, 123) derart an hinteren Enden des ersten und des zweiten Körpers (110, 120) gebildet sind, dass Finger eines Benutzers in die Griffe (113, 123) eingeführt werden, um das chirurgische Instrument zu halten.

13. Das chirurgische Instrument gemäß Anspruch 1, bei dem der erste und der zweite Körper (110, 120) durch einen Drehverbinder (115, 125) drehbar miteinander gekoppelt sind.

14. Das chirurgische Instrument gemäß Anspruch 13, bei dem der Drehverbinder (115, 125) folgende Merkmale aufweist:
einen ersten Drehverbinder (115), der einstückig in der Mitte des ersten Körpers (110) gebildet ist und ein Paar von Kopplungsvorsprüngen (115a, 115b) umfasst, die an dem Rand einer Seite des ersten Körpers (110) gebildet sind; und
einen zweiten Drehverbinder (125), der einstückig in der Mitte des zweiten Körpers (120) gebildet ist und ein Paar von Verbindungsrillen (125a, 125b) umfasst, die an dem Rand einer Seite gegenüber dem ersten Drehverbinder (115) derart gebildet sind, dass das Paar von Kopplungsvorsprüngen (115a, 115b) in das Paar von Verbindungsrillen (125a, 125b) eingeführt wird und sich der erste Drehverbinder (115) und der zweite Drehverbinder (125) in Bezug aufeinander in einem vorbestimmten Drehwinkel drehen.

## Revendications

1. Instrument chirurgical, comprenant:
un premier corps (110) comportant au moins un orifice d'aspiration (111) prévu à une extrémité avant de ce dernier;
un deuxième corps (120) configuré pour traverser le premier corps (110) et pour être couplé de manière rotative au premier corps (110);
une unité d'aspiration (200) prévue dans le premier corps (110), pour aspirer du sang d'un endroit saignant d'un patient à travers l'orifice d'aspiration (111) pendant la chirurgie; et
un illuminateur (300) prévu dans le deuxième corps (120), pour illuminer un espace entre les extrémités avant des premier et deuxième corps (110, 120),
dans lequel l'unité d'aspiration (200) comprend un tube d'aspiration (210) monté dans le premier corps (110), pour être couplé à l'orifice d'aspiration (111).

2. Instrument chirurgical selon la revendication 1, dans lequel un trou d'introduction de tube d'aspiration (110a) est formé dans le premier corps (110) dans le sens longitudinal, de sorte que le tube (210) d'aspiration soit introduit et monté dans le trou d'introduction de tube d'aspiration (110a).

3. Instrument chirurgical selon la revendication 2, dans lequel l'orifice d'aspiration (111) a un plus petit diamètre que le trou d'introduction de tube d'aspiration (110a).

4. Instrument chirurgical selon la revendication 1, dans lequel l'orifice d'aspiration (111) est formé sur une surface avant de l'extrémité avant ou sur une surface intérieure du premier corps (110).

5. Instrument chirurgical selon la revendication 1, dans lequel l'illuminateur (300) comprend une fibre optique (310) montée dans le deuxième corps (120) et configurée pour guider la lumière générée d'une source de lumière à irradier sur l'espace entre les extrémités avant des premier et deuxième corps (110, 120).

6. Instrument chirurgical selon la revendication 5, dans lequel un trou d'introduction de fibre optique (120a) est formé dans le deuxième corps (120) dans le sens longitudinal, de sorte que la fibre optique (310) soit introduite et montée dans le trou d'introduction de fibre optique (120a).

7. Instrument chirurgical selon la revendication 5, dans lequel au moins un trou d'illumination (121), à travers lequel est exposée une extrémité avant de la fibre optique (310), est formé dans le deuxième corps (120).

8. Instrument chirurgical selon la revendication 7, dans lequel le trou d'illumination (121) est formé sur une surface intérieure espacée vers l'arrière d'une distance prédéterminée de l'extrémité avant du deuxième corps (120).

9. Instrument chirurgical selon la revendication 7, dans lequel la fibre optique (310) exposée à travers le trou d'illumination (121) est disposée de manière à illuminer la zone centrale entre les extrémités avant ouvertes des premier et deuxième corps (110, 120).

10. Instrument chirurgical selon la revendication 1, dans lequel l'illuminateur est un bulbe lumineux ou une lampe DEL prévu sur une surface intérieure espacée vers l'arrière d'une distance prédéterminée de l'extrémité avant du deuxième corps (120), de manière à illuminer la zone centrale entre les extrémités avant ouvertes des premier et deuxième corps (110, 120).

11. Instrument chirurgical selon la revendication 1, dans lequel les extrémités avant des premier et deuxième corps (110, 120) sont recourbées vers le haut ou vers le bas.

12. Instrument chirurgical selon la revendication 1, dans lequel des poignées (113, 123) sont formées sur les extrémités arrière des premier et deuxième corps (110, 120), de sorte que les doigts d'un utilisateur soient introduits dans les poignées (113, 123), pour maintenir l'instrument chirurgical.

13. Instrument chirurgical selon la revendication 1, dans lequel les premier et deuxième corps (110, 120) sont couplés de manière rotative l'un à l'autre par un connecteur rotatif (115, 125).

14. Instrument chirurgical selon la revendication 13, dans lequel le connecteur rotatif (115, 125) comprend:
un premier connecteur rotatif (115) formé intégralement dans le centre du premier corps (110) et comportant une paire de saillies de couplage (115a, 115b) formées au bord d'un côté du premier corps (110); et
un deuxième connecteur rotatif (125) formé intégralement dans le centre du deuxième corps (120) et comportant une paire de rainures de connexion (125a, 125b) formées au bord d'un côté opposé du premier connecteur rotatif (115), de sorte que la paire de saillies de couplage (115a, 115b) soient introduites dans la paire de rainures de connexion (125a, 125b) et que le premier connecteur rotatif (115) et le deuxième connecteur rotatif (125) tournent l'un par rapport à l'autre selon un angle de rotation prédéterminé.
